# EUROPEAN PATENT SPECIFICATION

(11) **EP 0 619 718 B1**
(45) Date of publication and mention of the grant of the patent: **04.03.1998**
(21) Application number: 92924146.1
(22) Date of filing: 27.10.1992
(51) Int. Cl.: A61B 17/00

(54) **PLUG DEVICES FOR SEALING PUNCTURES**
VERSCHLUSSZAPFEN ZUM ABDICHTEN VON PUNKTIONSWUNDEN
DISPOSITIFS A TAMPON POUR L'OBTURATION D'ORIFICES DE PIQURE

(30) Priority: 28.10.1991 US 783645
(43) Date of publication of application: 19.10.1994
(73) Proprietor: KENSEY NASH CORPORATION, Exton, PA 19341 (US)
(72) Inventor: KENSEY, Kenneth, Chester Springs, PA 19425 (US); NASH, John, Dowingtown, PA 19335 (US)
(74) Representative: Shaw, Laurence
(86) International application number: US9209249
(87) International publication number: WO9308743

(56) References cited:
- WO-A-89/11301
- WO-A-92/05740
- US-A- 3 447 533
- US-A- 4 744 364
- US-A- 5 061 274

## Description

This invention relates generally to medical devices and more specifically to devices for sealing percutaneous openings or incisions in the body of a living being.

In United States patents 4,744,364 and 4,852,568, from which the first document is used as basis for the preamble of independent claims 1 and 12, there is disclosed a device for sealing an incision or puncture in tissue separating one portion of the body of a living being from another portion, e.g. a puncture in a blood vessel, duct or lumen, of a living being. The device basically comprises an elongated tubular body having an outlet at its distal end. The distal end of the device is arranged to be inserted, such as percutaneously, through the puncture. In the case where the puncture is an artery or other blood vessel, the outlet is inserted through the puncture so that it is located within the blood vessel's interior. An expandable closure is disposed within the device's tubular body and is formed so that it is held in a compact or compressed configuration within the tubular body. The tubular body also includes an ejector in the form of a plunger-like member arranged to force the closure out of the outlet into the portion of the being's body contiguous with the opening, e.g. within the interior of the blood vessel, whereupon the closure automatically expands to form an enlarged tissue engagement surface.

A retraction filament is secured to the closure to enable it to be pulled fully into the puncture after the device's tubular body has been withdrawn so that the engagement surface of the closure intimately engages the inner surface of the tissue contiguous with the puncture.

When the closure is used for sealing punctures or incisions in blood vessels it is constructed so that when it is open (i.e., in its expanded state) and in place sealing the puncture it doesn't appreciably block the flow of blood through the blood vessel.

In international application WO 89/11301 there is disclosed another device for sealing a similar puncture or incision. The device basically comprises a closure or plug formed of a material which when located within the puncture or incision expands automatically to engage the tissue contiguous therewith to seal the puncture and incision from the flow of body fluid therethrough. The closure disclosed in that application basically comprises a holding member, a filament, and a sealing member. The holding member is an elongated body, constructed like a toggle, and preferably formed of a biodegradable, thermoplastic polymer, such as polyglactide. The toggle is moulded onto the distal end of the filament. The filament is also biodegradable and, being flexible, enables the toggle to pivot to various orientations with respect to it. The sealing member basically comprises a cylindrical plug, preferably formed of a compressed foam, which is highly absorbent and which when disposed within the body swells in excess of its compressed diameter.

This swelling action effectively tilts the toggle from an original orientation parallel to the longitudinal axis of the insertion instrument. The instrument may then be withdrawn and the closure's filament retracted. This action pulls the closure's plug portion back through the puncture or incision in the artery wall until its toggle portion engages the inner surface of the artery wall to stop further retraction. As the toggle comes into engagement with the arterial wall, it effects the compression of the distal end portion of the sealing member. Moreover, the proximal end portion of the sealing member extends into the puncture or incision in the subcutaneous tissue to a point closely adjacent the skin. These actions effectively seal the puncture or incision from the passage of blood therethrough.

While the foregoing closures are generally suitable for their intended purposes, they still leave something to be desired from the standpoint of simplicity of construction and ease of use.

Accordingly, it is a general object of this invention to provide incision or puncture closure devices which overcome the disadvantages of the prior art.

It is a further object of this invention to provide simply constructed incision or puncture closure devices for quickly, easily, safely and effectively sealing a puncture or incision in tissue separating one portion of the body of a living being from another portion, e.g. for sealing a puncture or incision in an artery.

Accordingly the invention provides a closure device and a plug according to Claims 1 and 12 respectively below for sealing a small incision or puncture.

The closure device is arranged to be introduced through the incision or puncture by an introducer, e.g. an instrument having a tubular portion terminating in a free end through which the plug device may be expelled.

In one embodiment of the invention the plug means comprises a tube, e.g. is a rolled up sheet. At least the distal portion of the tube is formed of a non-hemostatic material. The tube has a central passageway. The retraction means comprises a filament which is connected to the distal portion of the tube adjacent the free end thereof and extends along the outside of the distal portion of said tube for a portion of the length of the tube and then passes into the central passageway of the tube and out the proximal portion thereof. This embodiment of the closure device is resistant to the formation of blood clots, and is of particular utility for sealing punctures or incisions in blood vessels, e.g. arteries, since the distal, non-hemostatic portion of the plug will not form any blood clot within the vessel.

In another embodiment of the invention the plug is also in the form of a tube, e.g. a rolled up sheet. In this embodiment the distal portion of the tube is also formed of non-hemostatic material. However at least a portion of the proximal portion of the tube is formed of a hemostatic material. The tube again has a central passageway and the retraction means is connected to the tube as described for the immediately preceding embodiment. This embodiment of the plug is particularly suited for sealing incisions or punctures in blood vessels due to the fact that the distal portion is non-hemostatic and thus will not produce blood clots. The hemostatic material at the proximal portion of the tube is arranged to be located within the track of the incision or puncture to serve as a means for anchoring the plug in position once the puncture or incision is sealed by the deformed distal end of the plug means. To best achieve that end the hemostatic material is also preferably expandable, e.g. is crushed collagen.

In yet another embodiment the plug means is in the form of a sheet which is pleated to form an elongated strip. The strip is then bent in two to form a bar-like member. The retraction means, e.g. filament, is connected to the apex of the bar-like member, with that apex forming the free end of the distal portion of the plug means. In accordance with this embodiment at least the distal portion of the plug means is formed of a non-hemostatic material. This embodiment of the plug is also resistant to the formation of blood clots, due to the non-hemostatic nature of its distal portion.

### Brief Description of the Drawings

Other objects and many of the attendant advantages of this invention will readily be appreciated as the same becomes better understood by reference to the following detailed description when considered in connection with the accompanying drawings wherein:
Fig. 1 is a side elevational view of a plug device;
Fig. 2 is a side elevational view, partially in section, showing an initial step of inserting the plug device shown in Fig. 1 into a portion of an instrument in preparation for its use;
Fig. 3 is a side elevational view, partially in section, similar to that of Fig. 1 and showing the final step readying the plug device and the instrument holding it for use to seal an incision or puncture.
Fig. 4 is a side elevational view, partially in section, showing a instrument having the plug device shown in Fig. 1 therein being inserted into a conventional sheath extending through a percutaneous incision or puncture into an artery to effect the sealing of that incision or puncture;
Fig. 5 is a side elevational view, partially in section, similar to Fig. 4 and showing an intermediate step in the process of sealing the incision or puncture;
Fig. 6 is a side elevational view, partially in section, similar to Fig. 4 and showing a subsequent intermediate step in the process of sealing the incision or puncture;
Fig. 7 is a side elevational view, partially in section, similar to Fig. 4 and showing a further subsequent intermediate step in the process of sealing the incision or puncture;
Fig. 8 is a side elevational view, partially in section, similar to Fig. 4 and showing the final step in the process of sealing the incision or puncture;
Fig. 9 is an isometric view of an embodiment of the closure device of this invention;
Fig. 10 is a side elevational view, partially in section, showing the closure device of Fig. 9 being inserted through a percutaneous incision or puncture into an artery to effect the sealing of that incision or puncture;
Fig. 11 is a side elevational view, partially in section, similar to Fig. 10 and showing an intermediate step in the process of sealing the incision or puncture;
Fig. 12 is a side elevational view, partially in section, similar to Fig. 10 and showing the final step in the process of sealing the incision or puncture;
Fig. 13 is an isometric view of still another embodiment of the closure device of this invention;
Fig. 14 is an isometric view showing the manner by which the closure device of Fig. 13 is formed;
Fig. 15 is a side elevational view, partially in section, showing the closure device of Fig. 13 being inserted through a percutaneous incision or puncture into an artery to effect the sealing of that incision or puncture;
Fig. 16 is a side elevational view, partially in section, similar to Fig. 15 and showing an intermediate step in the process of sealing the incision or puncture;
Fig. 17 is a side elevational view, partially in section, similar to Fig. 15 and showing the final step in the process of sealing the incision or puncture;
Fig. 18 is an isometric view of another embodiment of the closure device or plug of this invention;
Fig. 19 is a side elevational view, partially in section, showing the closure device or plug of Fig. 18 being inserted through a percutaneous incision or puncture into an artery to effect the sealing of that incision or puncture;
Fig. 20 is a side elevational view, partially in section, similar to Fig. 18 and showing an intermediate step in the process of sealing the incision or puncture; and
Fig. 21 is a side elevational view, partially in section, similar to Fig. 18 and showing the final step in the process of sealing the incision or puncture.

### Detailed Description of the Preferred Embodiment

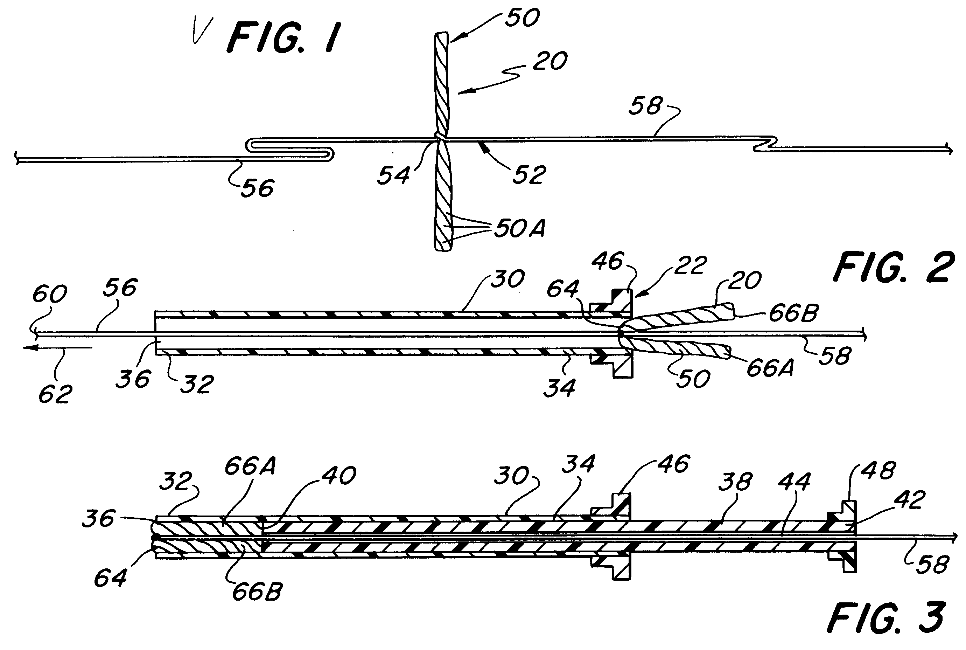

Referring now in greater detail to the various figures of the drawings wherein like reference characters refer to like parts, a closure or plug device is generally shown at 20 in Fig. 1. The device 20 is arranged to be used to effect the sealing of an incision or puncture or other small opening in any tissue separating two portions of the body of a living being to prevent liquid(s) or body fluid(s) to flow through the incision or puncture. The device 20 has particular utility when used in connection with intravascular procedures, such as angiographic dye injection, balloon angioplasty and other types of recanalizing of atherosclerotic arteries, etc. However, it is to be understood that while the description of the preferred embodiments of the closure (plug) devices contained herein is directed to the closing off of percutaneous incisions or punctures in arteries, such devices have much more wide-spread applications. Thus, the sealing of a percutaneous incision or puncture in an artery shown herein is merely exemplary.

In order to use the device 20 to seal the incision or puncture, the device is arranged to be located within an introducing instrument 22 like that shown in Fig. 3 and which will be described in detail later.

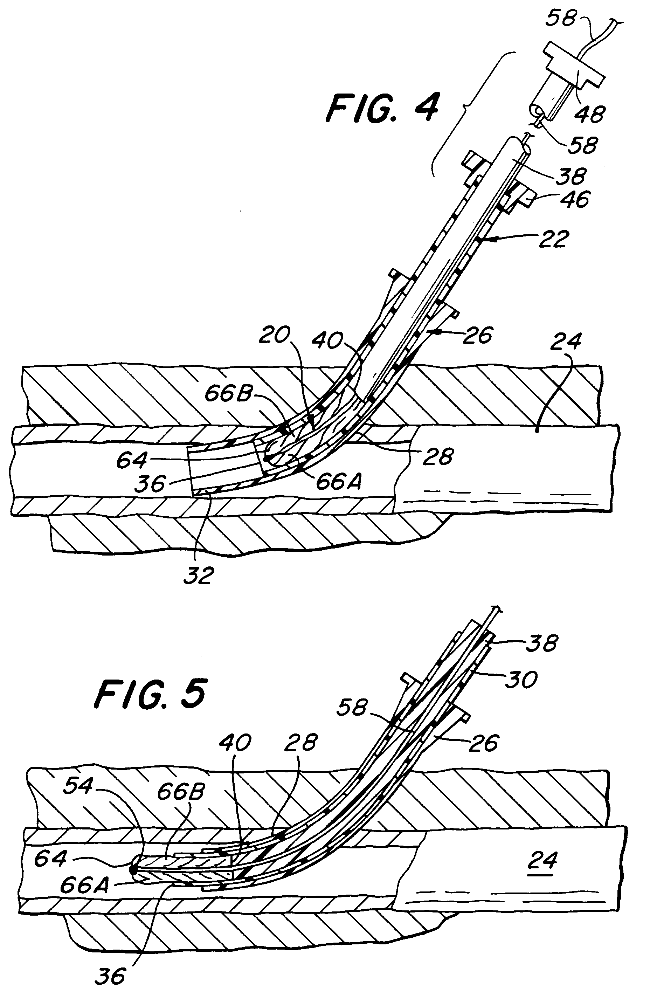

Before describing the device 20 and the instrument 22 for inserting it to seal the incision or puncture, a brief description of a typical, conventional, intravascular surgical procedure, e.g., catheter instrumentation of an artery, utilizing a percutaneous incision or puncture will be given to best appreciate the features of the invention. In such a procedure a cannula of an instrument, such as an angiographic needle (not shown), is inserted percutaneously through the skin into the artery, such as the femoral artery 24 at the situs for the instrument's 22 insertion (See Fig. 4). The needle cannula is held in place and the flexible end of a mini-guidewire (not shown) is then passed through the cannula into the artery to the desired depth (i.e., longitudinal position therealong). Once the mini-guide wire is in place the needle cannula is removed, leaving the guidewire in place. A conventional introducing sheath 26 and an arterial dilator (not shown) are then passed over the guidewire, through the puncture or incision 28 and into the artery 24. The guidewire and then the dilator are removed leaving the sheath in place. The catheter (not shown) or other intravascular instrument (not shown) is then inserted through the introducer sheath 26 and threaded down the artery to the desired intravascular location, e.g., the situs of the atherosclerotic occlusion. Once the intravascular procedure (e.g., angioplasty) has been completed, the catheter is removed. Thereafter, the sheath is removed and the surgeon or other trained person applies digital pressure to the percutaneous puncture until hemostasis has occurred.

The device 20 effects the hemostatic closure of the percutaneous (or any other type of puncture, incision or opening) in the artery or any other tissue separating two portions of the body without necessitating the application of pressure thereto. Thus, once the catheter or intravascular instrument has been removed, but with the introducer sheath 26 left in place, the instrument 22 holding the device 20 of the subject invention is inserted through the sheath, into the artery 24 and operated to expel the device 20 into the artery 24.

As can be seen clearly in Fig. 1, the device 20 basically comprises a closure or plug, whose details will be described later, which is arranged to be drawn into the puncture or incision 28 to seal it. The introducer sheath 26 is then removed and the closure or plug left in place. Due to its construction the closure or plug is ultimately absorbed by the surrounding tissue.

Referring now to Figs. 2 and 3, the details of instrument 22 will now be described. As can be seen the instrument 22 basically comprises a carrier in the form of a tubular body 30 having a distal end 32 and a proximal end 34. The distal end 32 forms a free end of the instrument and comprises an open outlet 36. The tubular body is preferably constructed of a sufficiently small outside diameter, e.g., 8F (French) and somewhat flexible material, such as polyethylene or polyvinyl chloride, to enable it to be inserted through the introducer sheath 26 into the artery 24, with the tubular body's outlet 36 within the artery 24 distally of the incision of puncture 28 as will be described later. A pusher member 38 is disposed within the tubular member 30. The pusher basically comprises an elongated, cylindrical rod-like member, having a free or distal end 40 and a proximal end 42. A central passageway 44 extends through the pusher. Preferably the pusher is also formed of a relatively flexible material, such as polyethylene or polyvinyl chloride, and is disposed within the interior of the tubular body as shown in Fig. 3 when the instrument is ready for use. The outside diameter of the pusher is slightly less than the inside diameter of the tubular body to enable the pusher to be manually moved, that is slid, down the longitudinal axis of the tubular body, to push or force the closure 20 out of the outlet 36, as will be described later.

The proximal end of the tubular member 30 includes a flange 46 and the proximal end 42 of the pusher 38 includes a similar flange forming a cap 48. The flanges 46 and 48 form portions to be gripped or engaged by the operator's fingers to enable the pusher to be moved (pushed) longitudinally down the tubular member to expel the closure 20, as will be described later.

Referring again to Fig. 1 it can be readily seen that the closure or plug 20 basically comprises a cord 50 and a filament 52. In accordance with one aspect of the preferred embodiment of the invention, the cord comprises a stranded yarn of plural fibers 50A of collagen or some other absorbable material. In particular one exemplary yarn consists of five strands 50A of .032 inch (.81 mm) fibers which are twisted together. The filament 52 is secured to the cord 50 by wrapping it about the midsection 54 of the cord and knotting it thereat. This action forms a pair of filament sections, namely, 56 and 58, with section 56 forming a distally extending section with portion 58 forming a proximately extending portion.

As can be seen clearly in Fig. 2 the plug 20 is arranged to be inserted into the tubular member 30 of the instrument 22 as follows: the distally extending filament portion 56 is inserted through the open proximal end of the tubular member 30 and extended therethrough until its free end 60 extends out of the outlet 36 of the tubular member. The distally extending filament portion 56 is then pulled in the distal direction, like shown by arrow 62, whereupon the cord 50 of the closure 20 enters into the open proximal end of the tubular member. This action causes the cord to fold in half to form a leading or apex portion 64 and a pair of trailing, i.e., proximately extending, wing portions 66A and 66B. The distally extending filament portion 56 is then continued to be pulled in the proximal direction, thereby drawing the folded plug 20 down the tubular member 30 until its apex 64 is immediately adjacent the open end 36 of the tubular member 30. When the plug is in this position (shown in Fig. 3) the proximally extending filament portion 58 extends in the proximal direction from the folded cord 50 through the tube 30 and out its open proximal end. The pusher member 38 is then inserted within the tubular member 30. In particular, the free end of the proximally extending filament portion 58 is introduced into the distally located opening in the central passageway 44 of the pusher and threaded down the central passageway until it extends out of the opening at the flange or head 48 of the pusher. The pusher is then introduced into the proximately located opening in the tubular member and slid down the interior thereof in the distal direction until its 40 free end is located immediately adjacent the wings 66A and 66B of the plug 20. The distally extending filament portion 56 is then cut off or severed from the apex 36 of the cord immediately adjacent the apex. Once this latter action has been achieved the instrument 22 is ready for use.

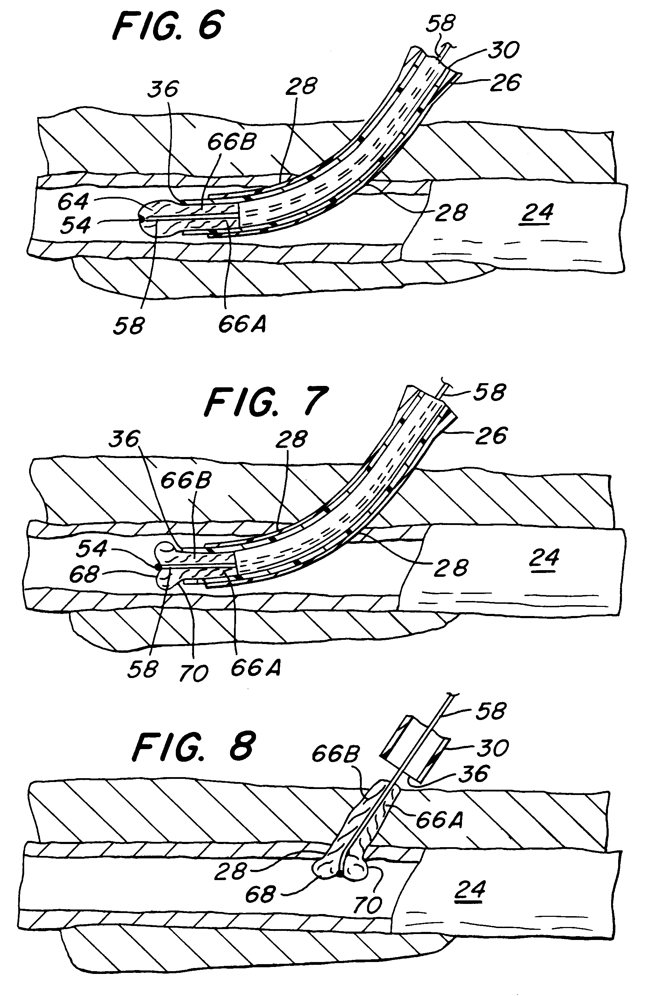

Operation of the instrument 22 is best understood by reference to Figs. 4-8 and is as follows: the instrument 22 is inserted within the introducer sleeve 26 so that the free end 32 of the tubular member 30 extends through the puncture or incision 28 like that shown in Fig. 4. The user then engages and pushes on the cap 48 of the pusher with his/her thumb while grasping the flange 46 of the tubular member between his/her fingers. This action slides the pusher in the distal direction within the tubular member 30 so that its free end 40 engages the end of the wing portions 66A and 66B of the plug to force the apex portion 64 of the plug out of the open free end 36 of the tubular member as shown in Fig. 5. The plug is left in this position for a few minutes, whereupon the portion extending into the artery expands slightly in the presence of the liquid, e.g., blood, etc., within the artery, as shown in Fig. 6.

The proximally extending filament portion 58 is then pulled in the proximal direction. This action causes the enlarged portion of the cord's apex to engage the free edge of the opening 36 of the tubular member 30 thereby further radially expanding and flattening that portion to form an enlarged or mushroom shaped head 68.

After the head 68 of the plug 20 has been expanded to its maximum diameter the introducer sleeve 26 is removed from the puncture or incision 28 and the instrument 22 is then withdrawn as shown in Fig. 8. This retraction action (i.e., the removal of the instrument from within the puncture or incision 28) causes the peripheral edge portions 70 on the underside of the enlarged head 68 of the plug to be drawn into close or intimate engagement with the tissue contiguous with the incision or puncture 28 to thereby seal that incision or puncture.

As can be seen in Fig. 8 with the closure in position the head 68 does not take up a substantial portion of the interior of the artery and thus does not block off or otherwise impede the flow of blood therethrough.

When the closure 20 of the subject invention is used to hemostatically seal a puncture or incision in an artery or other vessel, in order to minimize the risk of thrombosis the head of the closure which is exposed to the flow of blood through the artery may be coated with a non-thrombogenic material. Such a material can comprise a waxy coating, such as coconut oil, etc.

As mentioned earlier the cord 50 is formed of a resorbable, e.g., biodegradable, material. In accordance with the preferred embodiment of the invention, the filament 52 is also resorbable, and is preferably a suture of 3-0 size. These features enable the cord and filament to be left in place after hemostasis has occurred, since both will be absorbed by body's tissues thereafter. Accordingly, the plug does not have to be removed after having served its purpose.

Moreover, when the plug of the instant invention is used for sealing punctures or incisions in arteries a conventional clotting agent, such as tissue thromboplastin may be provided in the closure to accelerate hemostasis.

While the plug's cord has been described as comprising a stranded yarn of plural fibers such a construction is merely exemplary of various types of constructions. Thus, the "cord" may merely consist of a strip or bar of some resorbable material which is sufficiently flexible to fold over and form the heretofore described apex and wing portions.

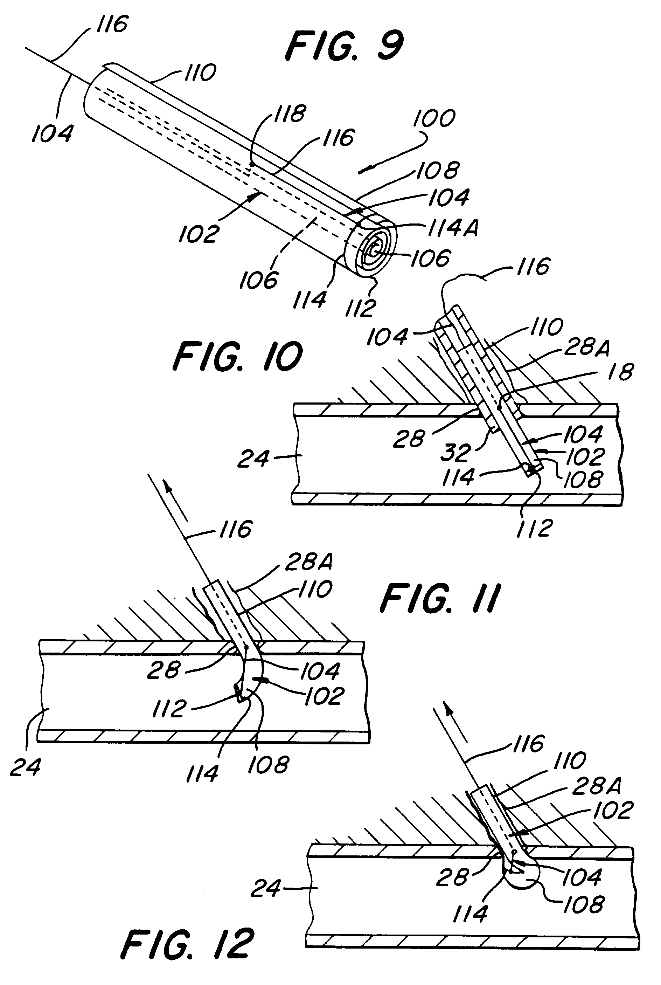

Referring now to Fig. 9 an embodiment of the closure device embodying the present invention is shown and designated by the reference number 100. As can be seen the closure device 100 basically comprises a plug member 102 and an associated retraction member 104. The retraction member comprises a filament or other strand-like member. The plug member 102 is a generally elongated member having a central passageway 106 extending longitudinally therethrough and has a distally located portion 108 and a proximally located portion 110. The free end of the distally located portion is designated by the reference number 112.

The plug member 102 may be formed in any manner so that it has a longitudinal passageway extending through at least the proximally located portion 110 to receive the filament 104 therethrough as will be described later. In the embodiment of Fig. 9 the plug member 102 is preferably formed of a sheet which is reeled up about a mandrel (not shown) to form a tube. Alternatively the plug may be formed of a rod or bar of material with a longitudinal passageway formed therein and extending through at least the proximally located portion of the rod or bar. Irrespective of the manner by which the plug member is formed, it is preferable that at least its distally located portion is formed of a non-hemostatic material, such as polyglycolic acid, polylactide, polylactic acid, non-thrombogenic collagen, or combinations thereof. Thus, in the embodiment shown in Fig. 9 the tubular member 102 is formed of a sheet of such material which has been rolled up.

The retraction filament 104 is also formed of any suitable, non-hemostatic material, e.g., conventional suture material, and is secured to the distally located portion 108 of the plug member 102 immediately adjacent the free end 112 thereof. In particular, the filament 104 includes a looped portion 114 and an extending portion 116. The looped portion 114 tightly encircles the periphery of the plug member 104 immediately adjacent the free end 112 and is knotted in place by a knot 114A. The extending portion 116 of the filament 104 lies longitudinally along the outer surface of the proximally located portion 110 of the plug member and passes into an opening or hole 118 in an intermediate portion thereof. The opening 118 communicates with the central longitudinal passageway 106. The remainder of the extending portion 116 of the filament 104 then passes through the central passageway 106 and exits at the top end of the plug member 104.

The distally located portion 108 of the plug member is deformable, e.g., bendable so that it can be bent back over itself. That action occurs once the closure device 100 is in place within the puncture or incision 28 to be sealed, e.g., the puncture or incision in the artery 24. In particular, the device 100 is introduced by any suitable introducer, e.g., a tubular introducer having an open free end 32 similar to that described heretofore, through the puncture or incision 28 so that the plug's distally located portion 110 is positioned within the interior of the artery and its proximal portion 108 is positioned within the puncture track 28A extending between the outer surface of the artery wall and the patient's skin. In this position the extending portion 116 of the filament 104 passes through incision or puncture track 28 to a point outside the body of the being as shown clearly in Fig. 10. The introducer is then removed from the body of the patient.

The externally located extending portion 116 of the filament 104 is then pulled away from the body, i.e., pulled in the proximal direction. Since the filament 104 is connected to the distally located portion of the plug member on the outer surface thereof and extends along that surface until it enters radially into the hole 118 the retraction or pulling on the filament 104 causes the distally located portion of the plug member contiguous with the free end to begin to bend as shown in Fig. 11. The continued pulling on the filament causes the free end 112 of the plug member 102 to bend back over itself and to engage the inner surface of the artery wall contiguous with the incision or puncture 28. This action has the effect of creating a somewhat enlarged head which prevents the plug from exiting the artery through the puncture or incision and which also effectively seals the puncture or incision so that blood cannot flow out of the artery through the puncture or incision.

As can be seen in Fig. 12 with the closure in position its enlarged head does not take up a substantial portion of the interior of the artery and thus does not block off or otherwise impede the flow of blood therethrough. Moreover, by virtue of the fact that the distally located portion of the plug means is non-hemostatic, the portion, e.g., head, of the plug means located within the interior of the artery will not produce any blood clot(s) therein.

In order to hold the closure device 100 in the position as shown in Fig. 12 any suitable holding or locking means can be utilized. Such means may take various forms and be located at various positions. For example, such means may comprise some component located on the skin of the being to hold the filament portion 116, or may comprise a component located within the puncture or incision track 28A to hold the proximally located portion 112 or the filament 116. Moreover, such holding or locking means may comprise a component located within the puncture track 28A to engage the exterior surface of the artery.

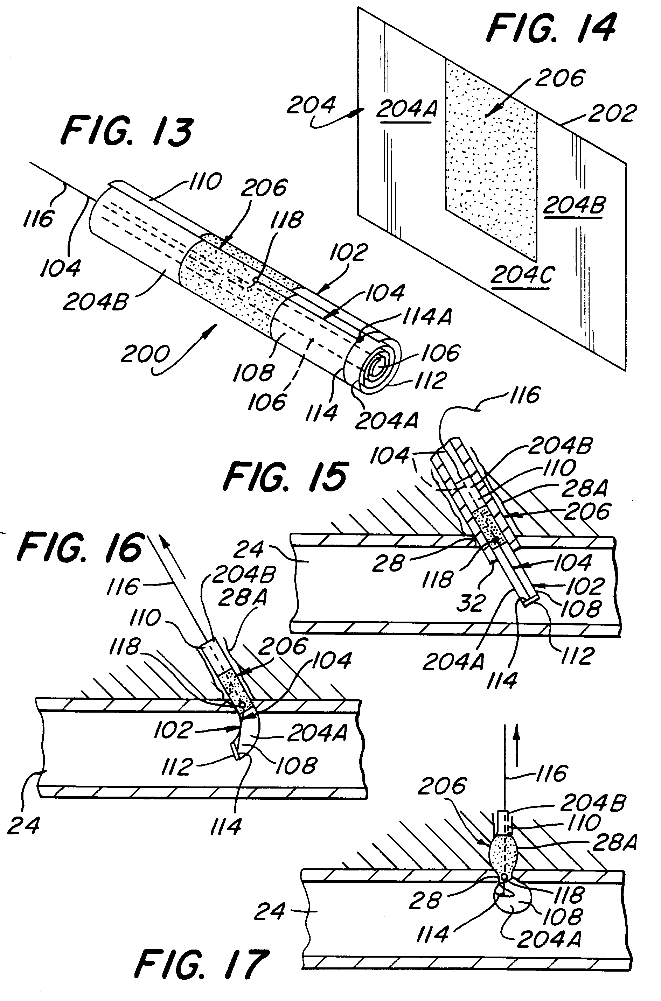

In Fig. 13 there is shown yet another alternative embodiment of the closure device of this invention. That closure device is designated by the reference number 200 and is similar in construction and operation to the closure device 100 except that it includes hemostatically operative means to hold or lock closure device 200 in position. In particular the device 200 includes means (to be described hereinafter) to hemostatically hold the proximal portion of the plug means within the incision or puncture track 28A once the incision or puncture in the artery has been sealed.

The construction of the closure 200 is similar to the closure 100. Moreover, the manner by which the sealing of the incision or puncture 28 is accomplished is the same with both of the closure devices 100 and 200. Thus, in the interest of brevity the various components which are common to both of those closure devices will be given the same reference numbers and their construction and operation will not be reiterated in detail hereinafter.

As can be seen in Fig. 13 the closure device 200 basically comprises a somewhat elongated plug member 102 and an associated retraction filament 104. The plug member 102 has a central passageway 106 extending longitudinally therethrough and includes a distally located portion 108 and a proximally located portion 110. The distally located portion 108 terminates in a free end 112.

The tubular plug member 102 of closure device 200 is formed of a sheet of a non-hemostatic material, such as polyglycolic acid, polylactide, polylactic acid, non-thrombogenic collagen, which is reeled up about a mandrel (not shown). However, unlike the sheet forming the plug member 102 of the closure device 100, the sheet forming the plug member 102 of the closure device 200 includes a portion which is hemostatic and a portion which is non-hemostatic. That sheet is shown in Fig. 14 and is designated by the reference number 202. Thus, as can be seen the sheet 202 includes a first section 204 formed of a non-hemostatic material and a second section 206 formed of a hemostatic material. The first section 204 of the sheet 202, when viewed in plan, is generally U-shaped having side portions 204A and 204B and an end portion 204C. The end portion forms what will be referred to later as the "inner" marginal edge of the sheet 202. The second section 206 of the sheet 202, when viewed in plan, is generally rectangular and is located within the confines of the portions 204A, 204B and 204C of the U-shaped section 204.

In accordance with a preferred embodiment of this invention the sheet section 204 is formed of polyglycolic acid, polylactide, polylactic acid, non-thrombogenic collagen, and the sheet section 206 is formed of an expandable material, e.g., crushed collagen.

The sheet 202 is rolled up starting with the inner marginal edge 204C. Thus, when the sheet 202 is completely rolled up the distally located portion 108 of the plug member 102 will be non-hemostatic (it will be formed by a portion 204A of section 204), whereas the intermediate portion of the proximally located portion of the plug member will be hemostatic (it will be formed by a portion of the section 206). The top of the proximally located portion of the plug member will be non-hemostatic (it will be formed by a portion 204B of the section 204). Thus, the hemostatic portion 206 will be held sandwiched between the non-hemostatic portions of the plug member.

Like the closure 100, the distally located portion 108 of closure 200 is deformable, e.g., bendable, so that it can be bent back over itself by pulling on the filament 104, like that described heretofore with reference to closure 100. Moreover, as can be seen in Figs. 15 - 17 the closure device 200 operates in the same manner as previously described, except for the locking or holding of the closure device 200 in place after the incision or puncture 28 in the wall of the artery 24 has been sealed. In this regard, as will be appreciated by those skilled in the art, blood which is in the tissue(s) contiguous with the puncture or incision track 28A will engage the expandable hemostatic material 206 of the plug member located within that track. This action will result in the expansion of the material 206 followed shortly thereafter by hemostasis, thereby locking the proximally located portion 110 of the plug means 200 in place.

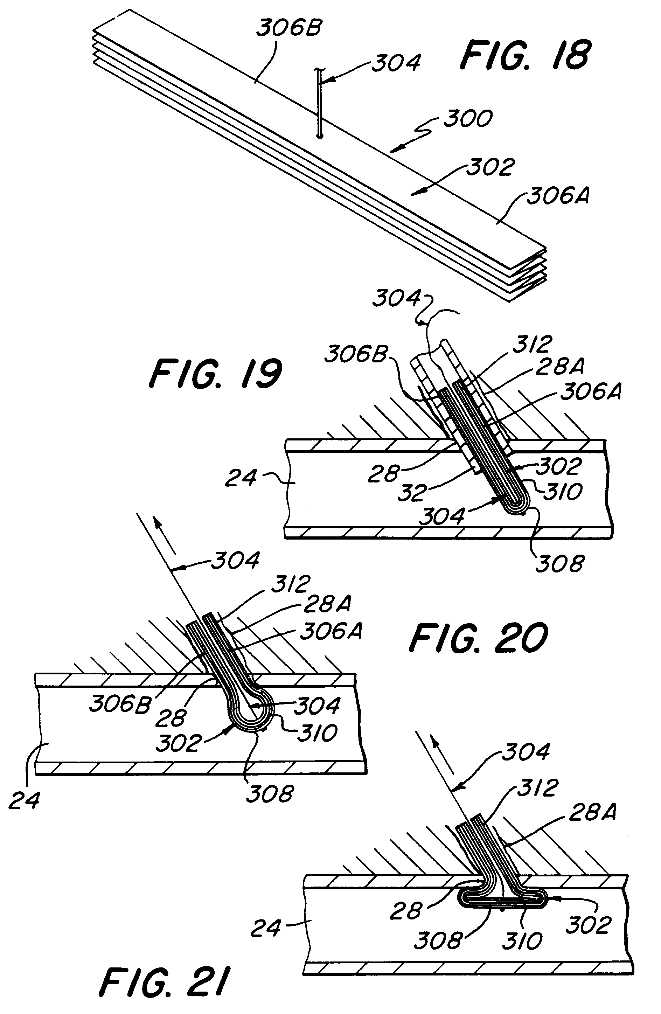

In Fig. 18 there is shown yet another embodiment of the closure device of this invention. That device is designated by the reference number 300 and basically comprises plug means 302 and associated retraction means 304. The plug means is formed from a sheet of material which is pleated to form an elongated rectangular strip. The strip is then bent in two to form a pair of legs 306A and 306B which extend parallel and immediately adjacent to each other and which together form a generally elongated plug member 306 The apex of the plug member 306 is designated by the reference number 308.

The portion of the plug member 306 contiguous with the apex 308 defines the distally located portion 310 of the plug member, with the apex 308 forming the free end of the distally located portion. The remaining portion of the member 306 comprises the proximally located portion 312.

The retraction means 304 comprises a filament similar to that described above. The distal end of the filament 304 is connected to the apex of the plug member 306. The remainder of the filament 304 extends between the two legs 306A and 306B of the plug member 306 beyond the top (proximal) end thereof.

Like the other closure device embodiments described above, the distally located portion 310 of the plug member 306 is deformable, e.g., bendable. It particular, it can be bent to form a generally enlarged, somewhat mushroom shaped, head upon the retraction or pulling of the filament 304. That action occurs once the closure device 300 is in place within the puncture or incision 28 to be sealed.

Thus, the closure device 300, like the devices 100 and 200, is introduced by any suitable introducer through the puncture or incision so that its distal portion 310 is located within the interior of the artery and its proximal portion 312 is located within the puncture track 28A extending between the outer surface of the artery wall and the patient's skin as shown in Fig. 19.

In this position the extending portion of the filament 304 passes through incision or puncture track 28 to a point outside the body of the being.

The introducer is removed and the externally located extending portion of the filament 304 is then pulled away from the body, i.e., pulled in the proximal direction. This action causes the distally located portion of the plug member contiguous with the free end to begin to bend into a loop configuration as shown in Fig. 20. Continued pulling on the filament causes the free end of the tubular member 30 to flatten out into a generally mushroom shaped head which engages the inner surface of the artery wall contiguous with the incision or puncture therein as shown in Fig. 21. This enlarged head has the effect of preventing the plug from exiting the artery through the puncture or incision and also effectively seals the puncture or incision so that blood cannot flow out of the artery through the puncture or incision.

Like the other embodiments of the closure devices described heretofore the enlarged head of the closure device 300 does not takes up a substantial portion of the interior of the artery and thus does not block off or otherwise impede the flow of blood therethrough. Moreover, like the closure devices 100 and 200 at least the distally located portion 310 of the plug means 302 is formed of a non-hemostatic material. In the embodiment shown herein, the entire member 302 is non-hemostatic, e.g., is formed of a sheet of polyglycolic acid, polylactide, polylactic acid, non-thrombogenic collagen. Thus, the head of the plug member which is located within the interior of the artery will not produce any blood clot(s) therein.

As should be appreciated from the foregoing the closure devices of the subject invention are very simple in construction and can be readily used to effectively and efficiently seal punctures or incisions in body organs or tissue, be they blood vessels, other lumens, ducts, etc. For example, the closure devices and their methods of use can be used for the purpose of sealing percutaneous transhepatic punctures to preclude the risk of bile leakage into the peritoneum, via the liver puncture site. Moreover, the devices and methods of use can be used for sealing percutaneous incisions in the lung or heart, such as could result from a wound.

Without further elaboration the foregoing will so fully illustrate our invention that others may, by applying current or future knowledge, adopt the same for use under various conditions of service.

## Claims

1. A closure device for sealing a small incision or puncture (28) in tissue separating a first internal portion of the body of a living being from a second internal portion thereof to prevent the flow of bodily fluid therebetween, said incision or puncture (28) comprising a track portion extending through said first internal portion, said closure device comprising plug means (102) and retraction means (104), characterised in that the plug means (102) comprises a generally elongated member having a central longitudinal axis, a distal portion (108) and a proximal portion (110), the distal portion (108) of the elongated member being deformable and including a free end (112), the retracting means (104) comprising a filament (104) having a first portion and a second portion, the first portion being secured to the elongate member adjacent the free end (112) thereof and laterally of said central longitudinal axis, the first portion of the filament (104) extending laterally of the central longitudinal axis of the elongated member along a first part of the distal portion of the elongated member for a portion of the length of the elongated member, to an intermediate portion, the second portion of the filament (104) extending along a second part of the elongated member and closer to the central longitudinal axis of the elongated member than the first portion of the filament (104), the second portion of the filament (104) extending between the intermediate portion and the proximal end of the elongated member and then out of the proximal portion of the elongated member, the distal portion of the elongated member being deformable so that when the filament (104) is pulled, the free end (112) of the elongated member is pulled toward the intermediate portion, thereby deforming the distal portion (108) so that it cannot pass back through the puncture (28).

2. The closure device of Claim 1, characterised in that the distal portion (110) of the plug is non-hemostatic.

3. The closure device of Claim 1 or 2, characterised in that the plug (102) comprises a tube, the tube having a central passageway (106), the second portion of the filament extending through the central passageway, the first portion of the filament extending along the outside of the distal portion (108).

4. The closure device of Claim 3, characterised in that the tube is formed of a rolled up sheet.

5. The closure device of any preceding Claim, characterised in that the distal portion (108) of the plug (102) is comprised of a material selected from the group consisting of polyglycolic acid, polylactide, polylactic acid, non-thrombogenic collagen.

6. The closure device of any preceding Claim, characterised in that the proximal portion (110) of the plug (102) comprises a hemostatic material to engage the body tissue contiguous with the track so that hemostasis occurs thereat to lock the closure device (102) in place.

7. The closure device of claim 6, characterised in that the hemostatic material is expandable.

8. The closure device of Claim 7, characterised in that the expandable material comprises crushed collagen.

9. The closure device of any of Claims 4 to 8, characterised in that the sheet (202) comprises a first portion (204) of generally U-shape viewed in plan and a second portion (206) disposed within an interior space generally defined by the interior of the generally U-shaped first portion (204), the generally U-shaped first portion (204) comprising non-hemostatic material and the second portion comprising hemostatic material, wherein when the sheet (202) is rolled up to form the tube, the hemostatic material is generally located between the distal (108) and proximal (110) portions of the tube.

10. The closure device of Claim 9, characterised in that the non-hemostatic material comprises polyglycolic acid and the expandable hemostatic material comprises crushed collagen.

11. The closure device of any one of Claims 1,2,3 and 6 to 9, characterised in that the plug (302) is formed of a sheet, the sheet being pleated to form a strip, the strip being bent in two at an apex (308) thereof to form the generally elongated member (306), the retracting means (304) comprising a filament secured to the apex (308) of the distal portion of the plug (302) forming the free end thereof, the closure device being positionable so that the distal portion (310) of the plug can be disposed within the first internal portion while the proximal portion (312) of the plug is disposed within the track, and with the filament extending to, and connected to the apex (308) so that when the filament is pulled the distal portion (310) of the plug (302) is deformed and it cannot pass back through the incision or puncture (28) and the incision or puncture (28) will be sealed by the deformed distal portion of the plug.

12. A plug for sealing an incision or puncture in tissue separating one portion of the body of a living being from another portion thereof to prevent the flow of bodily fluid from the one portion to the other portion through the incision or puncture, the plug being insertable into the incision or puncture by an instrument comprising a carrier, the carrier comprising a tubular member having a proximally located portion and a distally located portion, the distally located portion having an open free end arranged to be introduced through the incision or puncture, the proximately located portion being arranged to be located out of the body when the distally located portion is extended through the incision or puncture, characterised in that the plug (302) comprises a sheet material pleated into an elongated rectangular strip (302) and a thin filament (304), the strip (302) being folded in two to form an apex portion (308) and a pair of wing portions (306A, 306B) extending therefrom, the filament (304) being secured to the apex portion (308), the plug (302) being locatable within the tubular member so that the apex portion (308) is disposed adjacent the free end of the tubular portion and with the wing portions (306A, 306B) and a portion of the filament (304) extending towards the proximal portion, the plug (302) being partially expellable from the free end of the tubular member so that the apex portion (306) can extend through the incision or puncture (28), the filament (304) being drawable in the proximal direction to cause the apex portion (308) of the plug (302) to form an expanded head (310) having a tissue engagement surface, the plug thereby being arranged to be positioned so that the tissue engagement surface intimately engages the tissue contiguous with the incision or puncture (28).

## Patentansprüche

1. Verschluß zum Verschließen eines kleinen Einschnittes oder Einstiches (28) in Gewebe, daß einen ersten inneren Teil des Körpers eines Lebewesens von einen, zweiten inneren Teil trennt, um den Durchfluß von Körperflüssigkeit zwischen diesen zu verhindern, wobei der genannte Einschnitt oder Einstich (28) aus einem Spurteil besteht, der sich durch den genannten ersten inneren Teil erstreckt, wobei der Verschluß ein Stöpsel (102) und ein Rückzugelement (104) aufweist, dadurch gekennzeichnet, daß der Stöpsel (102) allgemein aus einen, länglichen Element besteht mit einer zentralen Längsachse, einem distalen Teil (108) und einem proximalen Teil (110), wobei der distale Teil (108) des länglichen Elements verformbar ist und ein freies Ende (112) aufweist, wobei das Rückzugelement (104) aus einem Faden (104) mit einem ersten und einem zweiten Teil besteht, wobei der erste Teil am länglichen Element neben dem freien Ende (112) desselben befestigt ist und seitlich von der genannten zentralen Längsachse, wobei sich der erste Teil des Fadens (104) seitlich zur zentralen Längsachse des länglichen Elements längs einem ersten Teil des distalen Teils des länglichen Elements über einen Teil der Länge des länglichen Elements bis zu einen, Zwischenteil erstreckt, wobei sich der zweite Teil des Fadens (104) über einen zweiten Teil des länglichen Elements erstreckt und näher zur zentralen Längsachse des länglichen Elements angeordnet ist, als der erste Teil des Fadens (104), wobei sich der zweite Teil des Fadens (104) zwischen dem Zwischenteil und dem proximalen Ende des länglichen Elements erstreckt und aus dem proximalen Teil des länglichen Elements hinaus, wobei der distale Teil des länglichen Elements so verformbar ist, daß wenn der Faden (104) gezogen wird, das freie Ende (112) des länglichen Elements auf den Zwischenteil zu gezogen wird, wobei der distale Teil (108) so verformt wird, daß er sich nicht zurück durch den Einstich (28) bewegen kann.

2. Verschluß gemäß Anspruch 1, dadurch gekennzeichnet, daß der distale Teil (110) des Stöpsels nicht haemostatisch ist.

3. Verschluß gemäß Anspruch 1 oder 2, dadurch gekennzeichnet, daß der Stöpsel (102) eine Röhre aufweist, wobei die genannte Röhre einen zentralen Kanal (106) aufweist, sich der zweite Teil des Fadens durch den genannten zentralen Kanal und sich der erste Teil des Fadens längs der Außenseite des distalen Teils (108) erstreckt.

4. Verschluß gemäß Anspruch 3, dadurch gekennzeichnet, daß die Röhre durch eine aufgerollte Folie gebildet wird.

5. Verschluß gemäß einem der vorstehenden Ansprüche, dadurch gekennzeichnet, daß der distale Teil (108) des Stöpsels (102) aus einem Material besteht, das aus einer Gruppe gewählt wird, die aus Polyglykolsäure, Polylaktid, Polylaktidsäure und nicht Thrombose verursachendem Collagen besteht.

6. Verschluß gemäß einem der vorstehenden Ansprüche, dadurch gekennzeichnet, daß der nahe Teil (110) des Stöpsels (102) aus einem haemostatischen Material besteht, um in das Körpergewebe einzugreifen, während er die Spur berührt, so daß an dieser Stelle Haemostase auftritt, um den Stöpsel (102) zu sichern.

7. Verschluß gemäß Anspruch 6, dadurch gekennzeichnet, daß das haemostatische Material dehnbar ist.

8. Verschluß gemäß Anspruch 7, dadurch gekennzeichnet, daß das dehnbare Material aus pulverisiertem Collagen besteht.

9. Verschluß gemäß einem der Ansprüche 4 bis 8, dadurch gekennzeichnet, daß die Folie (202) aus einem, bei Draufsicht, allgemein U-förmigen Teil (204) und einem zweiten Teil (206) besteht, der in einem Innenraum angeordnet ist, der allgemein vom Innern des allgemein U-förmigen ersten Teils (204) bestimmt wird, wobei der allgemein U-förmige erste Teil (204) aus nicht haemostatischem Material besteht und der zweite Teil aus haemostatischem Material, wobei sich das haemostatische Material, wenn die Folie (202) zu einer Röhre aufgerollt ist, allgemein zwischen den distalen (108) und proximalen (110) Teilen der Röhre befindet.

10. Verschluß gemäß Anspruch 9, dadurch gekennzeichnet, daß das nicht haemostatische Material aus Polyglykolsäure und das dehnbare haemostatische Material aus pulverisiertem Collagen besteht.

11. Verschluß gemäß jedem der Ansprüche 1, 2, 3 und 6 bis 9 dadurch gekennzeichnet, daß der Stöpsel (302) von einer Folie gebildet wird, wobei die Folie zu einem Streifen gefaltet wird und der Streifen am Scheitelpunkt (308) in zwei Teile gebogen wird, um das allgemein längliche Element (306) zu bilden, wobei das Rückzugelement (304) einen Faden aufweist, der an, Scheitelpunkt (308) des distalen Teils des Stöpsels (302) gesichert ist und das freie Ende desselben bildet, wobei der Verschluß so angeordnet werden kann, daß der distale Teil (310) des Stöpsels im ersten internen Teil angebracht werden kann, während der proximale Teil (312) des Stöpsels in der Spur angebracht wird und wobei sich der Faden bis zum Scheitelpunkt (308) erstreckt und mit diesem verbunden ist, so daß wenn der Faden gezogen wird, der distale Teil (310) des Stöpsels (302) verformt wird und sich nicht zurück durch den Einschnitt oder Einstich (28) bewegen kann und der Einschnitt oder Einstich (28) vom verformten distalen Teil des Stöpsels verschlossen wird.

12. Ein Stöpsel zum Verschließen eines Einschnittes oder Einstiches in Gewebe der einen Teil des Körpers eines Lebewesens von einem anderen Teil desselben trennt, um den Durchfluß von Körperflüssigkeit vom einen zum anderen Teil durch den Einschnitt oder Einstich zu verhindern, wobei der Stöpsel in den Einschnitt oder Einstich mit einem Instrument eingesetzt werden kann, das einen Träger enthält, der aus einem röhrenförmigen Element besteht mit einem proximal angeordneten und einem distal angeordneten Teil, wobei der distal angeordnete Teil ein offenes freies Ende aufweist, das so angeordnet ist, daß es durch den Einschnitt oder Einstich eingeführt werden kann, wobei der proximal angeordnete Teil so angeordnet ist, daß er sich wenn der distal angeordnete Teil durch den Einschnitt oder Einstich geführt wird, außerhalb des Körpers befindet, dadurch gekennzeichnet, daß der Stöpsel (302) aus einem Folienmaterial besteht, daß in längliche rechtwinklige Streifen (302) gefaltet wird und einem dünnen Faden (304), wobei der Streifen (302) in zwei Teile gefaltet wird, um einen Scheitelpunkt (308) zu bilden und zwei Flügelteile (306A, 306B), die von diesem auslaufen, der Faden (304) am Scheitelpunktteil (308) gesichert ist, der Stöpsel (302) im röhrenförmigen Element so angeordnet ist, daß der Scheitelpunktteil (308) neben dem freien Ende des röhrenförmigen Teils angeordnet ist, wobei die Flügelteile (306A, 306B) und ein Teil des Fadens (304) auf den proximalen Teil zulaufen, wobei der Stöpsel (302) teilweise aus dem freien Ende des röhrenförmigen Elements herausziehbar ist, so daß sich der Scheitelpunktteil (306) durch den Einschnitt oder Einstich (28) erstrecken kann, der Faden (304) in die proximale Richtung ziehbar ist, so daß der Scheitelpunktteil (308) des Stöpsels (302) einen dehnbaren Kopf (310) bildet mit einer Gewebe-Befestigungsfläche, wodurch der Stöpsel so angebracht wird, daß die Gewebe-Befestigungsfläche direkt am Gewebe im Anschluß an den Einschnitt oder Einstich (28) anliegt.

## Revendications

1. Un dispositif de fermeture pour sceller une petite incision ou perforation (28) dans le tissu séparant une première portion interne du corps d'un être vivant d'une deuxième portion interne de ce dernier pour empêcher l'écoulement du fluide corporel entre ces dernières, ladite incision ou perforation (28) comprenant une portion piste s'étendant à travers ladite première portion interne, ledit dispositif de fermeture comprenant un moyen bouchon (102) et un moyen de rétraction (104), caractérisé en ce que le moyen bouchon (102) comprend un membre généralement allongé ayant un axe longitudinal central, une portion distale (108) et une portion proximale (110), la portion distale (108) du membre allongé étant déformable et comportant une extrémité libre (112), le moyen de rétraction (104) comprenant un filament (104) ayant une première portion et une deuxième portion, la première portion étant fixée au membre allongé au droit de l'extrémité libre (112) de ce dernier et latéralement par rapport audit axe longitudinal central, la première portion du filament (104) s'étendant latéralement par rapport à l'axe longitudinal central du membre allongé le long d'une première partie de la portion distale du membre allongé sur une portion de la longueur du membre allongé, jusqu'à une portion intermédiaire, la deuxième portion du filament (104) s'étendant le long d'une deuxième partie du membre allongé et plus près de l'axe longitudinal central du membre allongé que la première portion du filament (104), la deuxième portion du filament (104) s'étendant entre la portion intermédiaire et l'extrémité proximale du membre allongé, puis sortant de la portion proximale du membre allongé, la portion distale du membre allongé étant déformable de sorte que, lorsque le filament (104) est tiré, l'extrémité libre (112) du membre allongé est tirée vers la portion intermédiaire, déformant ainsi la portion distale (108) de sorte qu'elle ne peut pas repasser par la perforation 28.

2. Le dispositif de fermeture de la revendication 1, caractérisé en ce que la portion distale (108) du bouchon est non-hémostatique.

3. Le dispositif de fermeture de la revendication 1 ou 2, caractérisé en ce que le bouchon (102) comprend un tube, le tube ayant un passage central (106), la deuxième portion du filament s'étendant à travers le passage central, la première portion du filament s'étendant le long de l'extérieur de la portion distale (108).

4. Le dispositif de fermeture de la revendication 3, caractérisé en ce que le tube est formé d'une feuille roulée.

5. Le dispositif de fermeture de toute revendication précédente, caractérisé en ce que la portion distale (108) du bouchon (102) comprend un matériau sélectionné à partir du groupe composé d'acide polyglycolique, de polylactide, d'acide polylactique et de collagène non-thrombogénique.

6. Le dispositif de fermeture de toute revendication précédente, caractérisé en ce que la portion proximale (110) du bouchon (102) comprend un matériau hémostatique pour engager le tissu corporel contigu à la piste de sorte que l'hémostase se produit en ce point pour verrouiller le dispositif de fermeture (102) en place.

7. Le dispositif de fermeture de la revendication 6, caractérisé en ce que le matériau hémostatique est expansible.

8. Le dispositif de fermeture de la revendication 7, caractérisé en ce que le matériau expansible comprend du collagène écrasé.

9. Le dispositif de fermeture de l'une quelconque des revendications 4 à 8, caractérisé en ce que la feuille (202) comprend une première portion (204) généralement en U (vue en-dessus) et une deuxième portion (206) disposée dans un espace intérieur généralement défini par l'intérieur de la première portion généralement en U (204), la première portion généralement en U (204) comprenant un matériau non-hémostatique et la deuxième portion comprenant un matériau hémostatique, dans quoi, lorsque la feuille (202) est roulée pour former le tube, le matériau hémostatique se trouve généralement entre les portions distale (108) et proximale (110) du tube.

10. Le dispositif de fermeture de la revendication 9, caractérisé en ce que le matériau non-hémostatique comprend de l'acide polyglycolique et en ce que le matériau hémostatique expansible comprend du collagène écrasé.

11. Le dispositif de fermeture de l'une quelconque des revendications 1, 2, 3, et 6 à 9, caractérisé en ce que le bouchon (302) est formé d'une feuille, la feuille étant plissée pour former une bande, la bande étant pliée en deux à un sommet (308) de cette dernière pour former le membre généralement allongé (306), le moyen de rétraction (304) comprenant un filament fixé au sommet (308) de la portion distale du bouchon (302) formant l'extrémité libre de cette dernière, le dispositif de fermeture étant positionnable de sorte que la portion distale (310) du bouchon puisse être disposée dans la première portion interne tandis que la portion proximale (312) du bouchon est disposée dans la piste, et le filament s'étendant jusqu'au sommet (308) et étant connecté à ce dernier de sorte que, lorsque le filament est tiré, la portion distale (310) du bouchon (302) est déformée et qu'elle ne peut pas repasser par l'incision ou perforation (28) et que l'incision ou perforation (28) sera scellée par la portion distale déformée du bouchon.

12. Un bouchon pour sceller une incision ou perforation dans le tissu séparant une portion du corps d'un être vivant d'une autre portion de ce dernier pour empêcher l'écoulement du fluide corporel de cette portion à l'autre portion à travers l'incision ou perforation, le bouchon étant insérable dans l'incision ou perforation à l'aide d'un instrument comprenant un support, le support comprenant un membre tubulaire ayant une portion située proximalement et une portion située distalement, la portion située distalement ayant une extrémité libre ouverte arrangée pour être introduite par l'incision ou perforation, la portion située proximalement étant arrangée pour être située hors du corps lorsque la portion située distalement est étendue à travers l'incision ou perforation, caractérisé en ce que le bouchon (302) comprend un matériau feuille plissé en une bande rectangulaire allongée (302) et un filament mince (304), la bande (302) étant pliée en deux pour former une portion sommet (308) et une paire de portions ailes (306A, 306B) s'étendant à partir de cette dernière, le filament (304) étant fixé à la portion sommet (308), le bouchon (302) pouvant être positionné dans le membre tubulaire de sorte que la portion sommet (308) est disposée au droit de l'extrémité libre de la portion tubulaire et les portions ailes (306A, 306B) et une portion du filament (304) s'étendant vers la portion proximale, le bouchon (302) étant partiellement expulsable de l'extrémité libre du membre tubulaire de sorte que la portion sommet (308) peut s'étendre à travers l'incision ou perforation (28), le filament (304) étant tirable dans le sens proximal pour faire en sorte que la portion sommet (308) du bouchon (302) forme une tête expansée (310) ayant une surface d'engagement avec le tissu, le bouchon étant, de ce fait, arrangé pour être positionné de sorte que la surface d'engagement avec le tissu s'engage intimement avec le tissu contigu à l'incision ou perforation (28).
